# EUROPÄISCHE PATENTSCHRIFT

(11) **EP 2 721 371 B1**
(45) Veröffentlichungstag und Bekanntmachung des Hinweises auf die Patenterteilung: **02.12.2020**
(21) Anmeldenummer: 12730189.3
(22) Anmeldetag: 13.06.2012
(51) Int. Cl.: G01B 11/25, A61C 13/00, G01B 9/02, G01B 11/24, G01B 21/00, A61B 5/00

(54) **VERFAHREN ZUR OPTISCHEN DREIDIMENSIONALEN VERMESSUNG EINES DENTALEN OBJEKTS**
METHOD FOR THE OPTICAL THREE-DIMENSIONAL MEASUREMENT OF A DENTAL OBJECT
PROCÉDÉ DE MESURE OPTIQUE TRIDIMENSIONNELLE D'UN OBJET DENTAIRE

(30) Priorität: 15.06.2011 DE 102011077564
(43) Veröffentlichungstag der Anmeldung: 23.04.2014
(73) Patentinhaber: Sirona Dental Systems GmbH, 64625 Bensheim (DE)
(72) Erfinder: PFEIFFER, Joachim, 64625 Bensheim (DE); THIEL, Frank, 64372 Ober-Ramstadt (DE)
(74) Vertreter: Özer, Alpdeniz
(86) Internationale Anmeldenummer: PCT/EP2012/061154
(87) Internationale Veröffentlichungsnummer: WO 2012/171935

(56) Entgegenhaltungen:
- EP-A1- 2 258 254
- EP-A2- 0 288 983
- EP-A2- 1 262 751
- WO-A1-2009/121922
- WO-A2-2004/085956
- WO-A2-2009/071611
- DE-A1- 10 302 055
- DE-A1- 19 944 021
- DE-A1-102007 005 726
- FR-A1- 2 635 965
- US-A- 4 511 252
- US-A- 5 471 303
- US-A1- 2002 080 366
- US-A1- 2007 194 214
- US-A1- 2010 253 773

## Beschreibung

### Technisches Gebiet

Die Erfindung betrifft ein Verfahren zur optischen dreidimensionalen Vermessung eines dentalen Objekts, wobei unter Verwendung eines ersten optischen dreidimensionalen Messverfahrens ein erster Bereich des dentalen Objekts vermessen wird, wobei zumindest an dem ersten Bereich eine Puderung erfolgt, und wobei erste Bilddaten erzeugt werden.

### Stand der Technik

Aus dem Stand der Technik sind mehrere Verfahren zur optischen dreidimensionalen Vermessung eines dentalen Objektes bekannt. Insbesondere sind optische dreidimensionale Verfahren, wie das Triagulationsverfahren, das konfokale Mikroskopierverfahren oder das Weißlichtinterferometrieverfahren bereits bekannt.

Aus DE 199 63 333 A1 ist ein weiteres optisches dreidimensionales Verfahren offenbart, wobei ein Farbmuster bekannter Struktur bestehend aus mehreren Musterelementen auf das Projekt projiziert wird. Dieses projizierte Farbmuster wird aufgenommen, wobei in einem weiteren Verfahrensschritt jedem der Musterelemente ein Projektionswinkel zugeordnet wird, mit dem sich bei bekannter Relativposition zwischen Kamera und Projektor die dreidimensionalen Koordinaten betrachteter Objektpunkte ermittelt werden können.

In WO 2004/010076 A1 ist eine Weiterentwicklung dieses Verfahrens offenbart, wobei die Projektionsdaten des Farbmusters unter Verwendung eines redundanten Codes codiert werden.

FR 2 635 965 A1 offenbart eine optische Vermessungsvorrichtung zur Verwendung eines Triangulationsverfahrens, wobei eine hochauflösende zweidimensionale, frei programmierbare Matrix-Lichtquelle verwendet wird. In einer Ausführungsform wird in einem ersten Modus ein erstes Projektionsmuster mit einer größeren Gittekonstante und im zweiten Modus ein zweites Projektionsmuster mit einer kleineren Gitterkonstante und damit einer höheren Auflösung projiziert. Die gröbere Messung wird verwendet, um die auftretenden Mehrdeutigkeiten zu beseitigen.

EP 1 262 751 A2 offenbart eine Vorrichtung zur Lichtanalyse, wobei eine dreidimensionale Vermessung einer Zahnoberfläche aus unterschiedlichen Richtungen unter Verwendung unterschiedlicher Lichtquellen durchgeführt wird. Dabei kann die Zahnoberfläche mit einem Kontrastpuder beschichtet werden, um möglichst geringe Reflexionen zu erhalten.

EP 2 258 254 A1 offenbart eine Vorrichtung zur dreidimensionalen Vermessung eines Zahns, wobei die Zahnoberfläche mit einem Pulver beschichtet werden kann, um Reflexionen zu vermindern und damit eine Registrierung von Farbdaten und von Geometriedaten zu.

WO 2004/085956 A2 offenbart ein Laser-Vermessungssystem zur dreidimensionalen Vermessung eines dentalen Objekts, wobei eine zu vermessende Zahnoberfläche mit einem Puder beschichtet werden kann, um Reflexionen zu.

WO 2009/071611 A2 offenbart eine Vorrichtung zur Aufnahme eines dentalen Aufnahmeobjekts unter Verwendung eines Streifenmusters, wobei die Zahnoberfläche mit einem Kontrastmittel in Form eines Pulvers beschichtet werden kann, um die Reflexionen an der Zahnoberfläche zu verhindern.

US 2010/0253773 A1 offenbart eine Vorrichtung zur dreidimensionalen Vermessung unter Verwendung eines Simulationsverfahrens, wobei verschiedene Farbfilter auf einer Scheibe angebracht sein können, die rotierbar gelagert ist.

EP 0 288 983 A2 offenbart eine Vorrichtung zur dreidimensionalen Vermessung eines Objekts unter Verwendung eines Triangulationsverfahrens, wobei unterschiedliche Projektionsmuster auf das Objekt abwechselnd projiziert werden, wobei auf einer Scheibe 122 eine erste Maske 32 für ein erstes Projektionsmuster und eine zweite Maske für ein zweites Projektionsmuster angeordnet sind, wobei durch die Rotation dieser Scheibe die Projektionsmuster abwechselnd auf das Objekt 64 projiziert werden.

US 4,511,252 offenbart eine Vorrichtung zur dreidimensionalen Vermessung eines Objekts, wobei abwechselnd mehrere Projektionsmuster auf das Objekt projiziert werden. In einer Ausführungsform sind auf einem flexiblen Filmstreifen mehrere Projektionsmasken angeordnet, wobei diese Projektionsmasken abwechselnd im Strahlengang der Vorrichtung gebracht werden können, um unterschiedliche Projektionsmuster, wie Streifenprojektionsmuster, auf das Objekt zu projizieren.

WO 2009/121922 A1 offenbart eine Vorrichtung zur optischen 3D-Vermessung, wobei in einem ersten Modus eine optische 3D-Vermessung mittels einer chromatischen konfokalen Messmethode mittels einer Triangulation-Messmethode und in einem zweiten Modus eine Farbmessung erfolgt. In einer Ausführungsform erfolgt die Umschaltung zwischen dem ersten Modus und dem zweiten Modus durch das Einbringen einer Blende 7 in den Strahlengang des Beobachtungsstrahls.

US 2007/0194214 A1 offenbart eine konfokale Vorrichtung zur optischen dreidimensionalen Vermessung eines dentalen Objekts.

US 5,471, 303 offenbart eine Vermessungsvorrichtung zur optischen dreidimensionalen Vermessung eines Objekts, umfassend einen ersten Modus unter Verwendung einer Weißlichtinterferiometrie-Vermessung und einen zweiten Modus unter Verwendung einer Phaseinterferiometrie-Vermessung.

DE 199 44 021 A1 offenbart eine Vorrichtung zur Vermessung eines Objekts unter Verwendung eines Weißlichtinterferiometrie-Messverfahrens.

US 2002/0080366 A1 offenbart eine Vorrichtung zur Vermessung eines Objekts unter Verwendung eines Weißlichtinterferiometrie-Messverfahrens.

DE 10 2007 005 726 A1 offenbart eine Vorrichtung zur optischen 3D-Vermessung unter Verwendung einer Weißlichtinterferiometrie-Messmethode.

DE 103 02 055 A1 offenbart eine Vorrichtung zur dreidimensionalen Vermessung eines Objekts unter Verwendung einer tiefen Abtastung mittels einer Weißlichtinterferiometrie-Messmethode.

Bei dem bekannten Streifenprojektionsverfahren wird das Messobjekt aus parallelen hellen und dunklen Streifen unterschiedlicher Breite beleuchtet. In einem weiteren Schritt wird das projizierte Streifenmuster unter einem bekannten Blickwinkel zur Projektion mittels einer Kamera aufgenommen. Unter Verwendung eines sogenannten Phasenschiebeverfahrens wird eine Projektionskoordinate bestimmt, die die Nummer des Streifens wiedergibt. Die Nummer des Streifens im Projektor entspricht einer Bildkoordinate in der Kamera. Bei einer bekannten Kameraposition und einer bekannten Projektorposition relativ zur Objekt kann der Schnittpunkt, der durch den jeweiligen Streifen spezifiziert ist, und der Geraden, die durch die Koordinate in der Kamera spezifiziert ist, berechnet werden. Für jeden der Messpunkte wird auf diese Weise die dreidimensionale Koordinate der Oberfläche bestimmt.

Bei der Weißlichtinterferometrie wird ein Licht geringer Kohärenzlänge verwendet, so dass farbige Indifferenzen entstehen, wenn die Weglängen im Referenz- und Objektstrahl nahezu gleich sind. Beim Verändern der Weglänge wird das Interferenzmuster verändert, so dass anhand des Interferenzmusters der Abstand zur Oberfläche des Messobjekts bestimmt werden kann.

Bei dem dreidimensionalen konfokalen Mikroskopieverfahren wird die Oberfläche des dentalen Objekts schrittweise abgetastet, wobei eine Fokalebene schrittweise verschoben wird. Das Licht außerhalb der Fokalebene wird mittels einer Lochblende möglichst ausgeblendet. Aus den gemessenen Bilddaten der einzelnen Schritte unterschiedlicher Fokalebenen kann anschließend ein dreidimensionales Modell des vermessenen Objekts berechnet werden.

Ein Nachteil des Streifenprojektionsverfahrens ist, dass für eine präzise Aufnahme eine nicht spiegelnde Oberfläche des vermessenen Objekts Vorraussetzung ist. Dafür wird das dentale Objekt meist mit einem speziellen Puder vor der Aufnahme beschichtet. Nach der Aufnahme wird die aufgetragene Puderschicht entfernt.

Bei einer fehlenden Puderung wird lediglich eine geringe Genauigkeit erreicht, da Aufnahmefehler durch ungleichmäßige Reflexionen erzeugt werden.

Die übrigen genannten dreidimensionalen optischen Verfahren ohne vorangehende Puderung bieten für bestimmte Anwendungen, wie die Vermessung eines Präparationsgebiets zur Planung einer dentalen Restauration, eine nur unzureichende Genauigkeit.

Es gibt jedoch Messaufgaben für die nicht die höchstmögliche Genauigkeit bei der Vermessung erforderlich ist. Solche Messaufgaben sind zum Beispiel die Vermessung des gesamten Kiefers, die Vermessung der Gegenzähne eines Präparationsgebietes und eine Übersichtsaufnahmen.

Die Aufgabe der vorliegenden Erfindung besteht daher darin ein Verfahren zur optischen dreidimensionalen Vermessung eines dentalen Objekts bereitzustellen, wobei eine schnelle und einfache Vermessung bei einer ausreichenden Genauigkeit die jeweilige Messaufgabe ermöglicht wird.

### Darstellung der Erfindung

Die Erfindung betrifft ein Verfahren zur optischen dreidimensionalen Vermessung eines dentalen Objekts, wie im Anspruch 1 beansprucht, wobei unter Verwendung eines ersten optischen dreidimensionalen Messverfahrens ein erster Bereich des dentalen Objekts vermessen wird. Das erste optische dreidimensionale Messverfahrens beruht dabei auf einem Triangulationsverfahren und auf einem Streifenprojektionsverfahren. Vor der Vermessung erfolgt an dem ersten Bereich eine Puderung, wobei erste Bilddaten erzeugt werden. Anschließend wird unter Verwendung eines zweiten optischen dreidimensionalen Messverfahrens, das weniger präzise als das erste optische dreidimensionale Messverfahren ist, ein zweiter Bereich des dentalen Objekts ohne vorangegangene Puderung vermessen, wobei zweite Bilddaten erzeugt werden. Die ersten Bilddaten werden danach mit den zweiten Bilddaten zu einer überlagerten dreidimensionalen Aufnahme zusammengeführt.

Das erste optische dreidimensionale Messverfahren ermöglicht eine präzise Vermessung des dentalen Objekts bei möglichst geringen Abweichungen von den tatsächlichen Abmessungen des Objekts und kann beispielsweise das trigonometrische Streifenprojektionsverfahren mit vorangehender Puderung des Objekts sein. Der erste Bereich erfordert eine präzise Vermessung und kann beispielsweise eine Präparation und Nachbarzähne dieser Präparation zum Einsetzen einer geplanten Restauration umfassen. Die ersten Bilddaten beinhalten eine dreidimensionale Struktur des ersten Bereichs des dentalen Objekts und können in einem Speicher abgelegt sein. Das zweite optische dreidimensionale Messverfahren weist zwar eine geringere Präzision auf, kann jedoch auf eine einfachere und schnellere Art und Weise als das erste Messverfahren durchgeführt werden. Die Vorteile des zweiten Messverfahrens gegenüber dem ersten Messverfahren können sein, dass das zweite Messverfahren weniger empfindlich gegenüber Verwacklungen des Handstücks beim Vermessen ist, dass eine Puderung des Objekts zur Verhinderung störender Reflexionen nicht erforderlich ist, dass bei der Vermessung Farbinformationen des Objekts gewonnen werden können und dass die Vermessung kontinuierlich während einer Überflugbewegung (on-the-fly-fähig) über das zu vermessende Objekt und nicht schrittweise durch mehrere einzelne Aufnahmen aus unterschiedlichen Raumwinkeln erfolgt. Die erzeugten zweiten Bilddaten können ebenfalls in einem Speicher abgelegt werden. Anschließend werden die ersten Bilddaten mit den zweiten Bilddaten mittels eines Computers zu einer überlagerten dreidimensionalen Aufnahme zusammengeführt, die eine erforderliche Präzision am ersten Bereich und am zweiten Bereich des aufgenommenen dentalen Objekts aufweist.

Ein Vorteil dieses Verfahrens ist, dass eine für die spezielle Messaufgabe optimierte schnelle, einfache und den entsprechenden Genauigkeitsanforderungen dieser Messaufgabe genügende dreidimensionale Vermessung ermöglicht wird.

Ein weiterer Vorteil dieses Verfahrens ist, dass beim Zusammenführen der beiden Bilddaten Abweichungen zwischen den ersten Bilddaten und den zweiten Bilddaten und damit optische Fehler registriert werden, die anschließend korrigiert werden können.

Beim ersten Messverfahren wird durch die Puderung der erste Bereich, der eine Präparationsstelle sein kann, in einem ersten Schritt mit einem Puder gegen Reflexionen beschichtet und in einem zweiten Schritt mittels eines dentalen Handstücks, das auf dem Streifenprojektionsverfahren beruht, vermessen. Zur Verbesserung der Präzision kann beim ersten präzisen Messverfahren kurzwelliges blaues Licht verwendet werden, um die Aufnahmegenauigkeit zu verbessern. Vorzugsweise kann ein blaues Licht mit einer Wellenlänge von 400 nm bis 500 nm verwendet werden.

Der zweite Bereich wird mittels des zweiten Messverfahrens vermessen, welches im Bezug auf Reflexionen weniger empfindlich ist, so dass der zweite Bereich ohne einer vorangehenden Puderung auskommt.

Vorteilhafterweise kann das zweite optische dreidimensionale Messverfahren dem ersten optischen dreidimensionalen Messverfahren entsprechen mit dem Unterschied, dass eine Gitterperiode eines Projektionsgitters größer ist und keine Puderung des zweiten Bereichs erfolgt.

Das zweite Messverfahren beruht damit, wie das erste Messverfahren, auf dem Streifenprojektionsverfahren, wobei jedoch die zweite Gitterperiode des Projektionsgitters beim zweiten Verfahren größer als die erste Gitterperiode des Projektionsgitters beim ersten Verfahren ist und keine Puderung des zweiten Bereichs erforderlich ist. Dadurch ist das zweite Messverfahren zwar weniger präzise aber einfacher in der Durchführung. Die erste Gitterperiode des Projektionsgitters kann zwischen 300 µm und 400 µm, vorzugsweise 350 µm, und die zweite Gitterperiode des Projektionsgitters kann zwischen 600 µm und 800 µm, vorzugsweise 700 µm, betragen.

Vorteilhafterweise kann das zweite optische dreidimensionale Messverfahren auf einem konfokalen Mikroskopieverfahren beruhen.

Das konfokale Mikroskopieverfahren zur dreidimensionalen Vermessung ist weniger empfindlich bezüglich Spiegelungen an der Oberfläche des Objekts als das Streifenprojektionsverfahren und ermöglicht somit eine dreidimensionale Formerfassung ohne Puderung des Objekts. Somit kann der zweite Bereich unter Verwendung des konfokalen Mikroskopieverfahrens ohne Puderung vermessen werden. Dabei kann auch die Farbe des Objekts erfasst werden. Der Nachteil dieses Verfahrens ist, dass die Auflösung, die von den Abmessungen der Konfokalebene abhängig ist, geringer als beim Streifenprojektionsverfahren ist und dass die Dauer der Vermessung deutlich länger ist als beim Streifenprojektionsverfahren.

Vorteilhafterweise kann das zweite optische dreidimensionale Messverfahren auf einem Weisslichtinterferometrieverfahren beruhen.

Dadurch kann der zweite Bereich mittels eines dentalen Handstücks, das auf dem Weißlichtinterferometrieverfahren beruht, vermessen werden. Das Weißlichtinterferometrieverfahren ist weniger empfindlich bei Spiegelungen an der Oberfläche des Objekts und ermöglicht somit eine dreidimensionale Formerfassung des zweiten Bereichs ohne Puderung. Zusätzlich zur dreidimensionalen Vermessung können auch Farbinformationen der Oberfläche des Objekts gewonnen werden.

Vorteilhafterweise kann das zweite optische dreidimensionale Messverfahren auf einem Triangulationsverfahren mit farblichen Mustern beruhen.

Das bekannte Triagulationsverfahren mit farblichen Mustern hat den Vorteil, dass die Vermessung keiner vorangehenden Puderung des zweiten Bereichs bedarf und dass zusätzliche Farbinformationen des dentalen Objekts gewonnen werden können.

Vorteilhafterweise können bei Durchführung des zweiten optischen dreidimensionalen Messverfahrens Farbinformationen des dentalen Objekts erzeugt werden.

Dadurch können zusätzlich zu den dreidimensionalen Abmessungen des vermessenen Objekts Farbinformationen des Objekts gewonnen werden. Diese Farbinformationen des zweiten Bereichs, beispielsweise der Nachbarzähne, können zur Planung der Restauration verwendet werden, um den Farbton der geplanten Restauration an den Farbton der Nachbarzähne anzupassen.

Vorteilhafterweise kann der erste Bereich des dentalen Objekts eine Präparationsstelle in der Mundhöhle eines Patienten sein.

Dadurch umfasst der erste mit dem ersten präziseren Messverfahren aufgenommene Bereich die Präparationsstelle zum Einsetzen einer Restauration. Die Nachbarzähne, die gegenüberliegenden Zähne, der restliche Kieferbereich und/oder das die Präparationsstelle umgebende Zahnfleisch können anschließend mittels des zweiten weniger präzisen Messverfahrens vermessen werden.

Vorteilhafterweise kann der zweite Bereich des dentalen Objekts einen Gegenzahn, einen zum ersten Bereich benachbarten Nachbarzahn oder einen gesamten Kiefer umfassen.

Dadurch werden die genannten Strukturen mit einer geringeren Präzision erfasst und dienen lediglich zur Orientierung und Planung der einzusetzenden Restauration, die sowohl in ihrer Form und/oder in ihrer Farbgestaltung an die Nachbarzähne und die Gegenzähne angepasst wird.

Vorteilhafterweise kann der zweite Bereich den ersten Bereich des dentalen Objekts umfassen.

Dadurch wird der erste Bereich sowohl mittels des ersten Messverfahrens als auch mittels des zweiten weniger präzisen Messverfahrens erfasst.

Vorteilhafterweise können die ersten Bilddaten mit den zweiten Bilddaten verglichen werden und dadurch fehlerhafte Stellen in den ersten Bilddaten erkannt werden.

Dadurch können beispielsweise optische Fehler, die durch fehlerhafte Puderung des ersten Bereichs verursacht wurden erkannt werden. Eine Verwacklung des verwendeten Handstücks während der Vermessung des ersten Bereichs mittels der ersten Messmethode kann ebenfalls zu Aufnahmefehlern führen, die durch den Vergleich mit den zweiten Bilddaten erkannt werden können.

Vorteilhafterweise können die fehlerhaften Stellen in den ersten Bilddaten korrigiert werden.

Die Korrektur der fehlerhaften Stellen kann automatisiert unter Verwendung bekannter Mustererkennungsalgorithmen oder manuell durch den Benutzer erfolgen. Das Ergebnis der Korrektur ist eine fehlerlose Aufnahme, die sowohl den ersten als auch den zweiten Bereich umfasst.

Vorteilhafterweise können der zweite Bereich und der erste Bereich sich nicht überschneiden.

Dadurch wird der erste Bereich des Objekts nur unter Verwendung der ersten Messmethode vermessen.

Ein Beispiel, das nicht im Anwendungsbereich der vorliegenden Ansprüche fällt, ist eine Vermessungsvorrichtung zur optischen dreidimensionalen Vermessung eines dentalen Objekts, umfassend erste Mittel zur Verwendung eines ersten präzisen optischen dreidimensionalen Messverfahrens, das auf einem Triangulationsverfahren und auf einem Streifenprojektionsverfahren beruht. Die ersten Mittel umfassen ein erstes Projektionsgitter mit einer ersten Gitterkonstante. Die Vermessungsvorrichtung umfasst zusätzlich zweite Mittel zur Verwendung eines zweiten weniger präzisen optischen dreidimensionalen Messverfahrens. Die Vermessungsvorrichtung umfasst darüber hinaus einen Schalter und Verstellmittel, um die Vermessungsvorrichtung zwischen einem ersten Modus zum Betrieb nach dem ersten Verfahren und einem zweiten Modus zum Betrieb nach dem zweiten Verfahren zu schaltet. Beim Umschalten vom ersten Modus in den zweiten Modus mittels der Verstellmittel werden die zweiten Mittel anstelle der ersten Mittel im Strahlengang der Vermessungsvorrichtung angeordnet.

Die Vermessungsvorrichtung ermöglicht die Durchführung des oben genannten erfinderischen Verfahrens. Im ersten Modus kann das dentale Objekt unter Verwendung des ersten präzisen optischen Messverfahrens und im zweiten Modus unter

Verwendung des zweiten weniger präzisen optischen Messverfahrens vermessen werden. Abhängig vom eingestellten Modus werden die ersten Mittel oder die zweiten Mittel in den Strahlengang der Vermessungsvorrichtung eingebracht. Das erste Projektionsgitter kann unterschiedliche Projektionsmuster, wie parallele Streifen, aufweisen. Der Schalter kann manuell durch einen Benutzer betätigbar sein.

Ein Vorteil der Vermessungsvorrichtung ist, dass das dentale Objekt wahlweise unter Verwendung des ersten Messverfahrens oder des zweiten Messverfahrens mit derselben Vermessungsvorrichtung erfasst werden kann. Dadurch ist ein Wechsel der Vermessungsvorrichtung nicht erforderlich, so dass die Dauer der gesamten Vermessung verkürzt wird.

Die zweiten Mittel sind zur Verwendung eines konfokalen Mikroskopieverfahren geeignet und umfassen eine Lichtquelle, eine erste Lochblende, eine zweite Lochblende, einen Strahlteiler und/oder einen Detektor. Die zweiten Mittel sind im zweiten Modus derart angeordnet, dass ein von der Lichtquelle abgestrahlter Beleuchtungsstrahl die erste Lochblende durchstrahlt und mittels Fokussierungsmittel auf eine zu vermessende Fokusebene fokussiert wird. Die zweite Lochblende ist so angeordnet, dass ein vom Objekt in der Fokusebene zurückgestreute Beobachtungsstrahl durch die zweite Lochblende zum Detektor gelangt und die Beobachtungsstrahlen außerhalb der Fokusebene ausgeblendet werden. Dadurch wird im zweiten Modus eine Vermessung mittels des konfokalen Mikroskopieverfahrens durchgeführt, welches neben der Erfassung der dreidimensionalen Struktur auch die Erfassung der Farbe des dentalen Objekts ermöglicht.

### Kurzbeschreibung der Zeichnungen

Die Erfindung wird anhand der Zeichnungen erläutert. Es zeigt, die
- Fig. 1: eine Skizze einer Vorrichtung zur Durchführung des vorliegenden Verfahrens mit zwei Handstücken;
- Fig. 2: eine Skizze einer Vorrichtung zur Durchführung des vorliegenden Verfahrens mit einem Handstück;
- Fig. 3: eine Skizze einer ersten Vermessungsvorrichtung aus Fig. 2;
- Fig. 4: eine Skizze einer zweiten Vermessungsvorrichtung aus Fig. 2;
- Fig .5: eine Skizze einer dritten Vermessungsvorrichtung aus Fig. 2.

### Ausführungsbeispiel

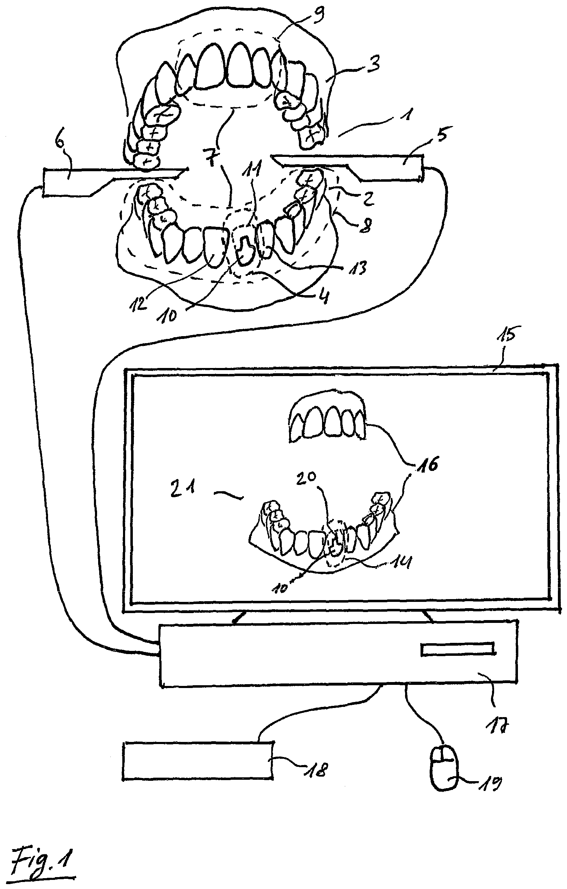

Die Fig. 1 zeigt eine Skizze zur Verdeutlichung des vorliegenden Verfahrens zur optischen dreidimensionalen Vermessung eines dentalen Objekts 1, das einen Unterkiefer 2 und einen Oberkiefer 3 umfasst. Das dentale Objekt 1 kann auch nur Teile des Unterkiefers 2 oder des Oberkiefers 3 umfassen. Ein erster Bereich 4, der durch eine gestrichelte Linie umrandet ist, wird unter Verwendung einer ersten präzisen Messvorrichtung 5 vermessen, die zur Durchführung eines ersten präzisen optischen dreidimensionalen Messverfahrens geeignet ist. Die erste Messvorrichtung 5 kann ein dentales Handstück sein, wobei das erste Messverfahren auf einem Triangulationsverfahren und auf einem Streifenprojektionsverfahren beruht. Vor der Vermessung mittels der ersten Messvorrichtung 5 wird der erste Bereich 4 mit einem Puder beschichtet, das Lichtreflexionen zur Verbesserung der Messgenauigkeit verhindert. Anschließend wird mittels einer zweiten weniger präzisen Messvorrichtung 6 ein zweiter Bereich 7 des dentalen Objekts 1 vermessen, der die gesamte untere Zahnreihe 8 und Gegenzähne 9 umfasst. Der erste Bereich 4 kann beispielsweise eine Präparation 10 für eine herzustellende Restauration 11 umfassen. Der erste Bereich 4 muss präzise vermessen werden, um eine genaue Passung der Restauration 11 an die Präparation 10 zu gewährleisten. Die zweite Messvorrichtung 6 ist zur Verwendung eines weniger präzisen zweiten optischen dreidimensionalen Messverfahrens geeignet. Das zweite Messverfahren kann beispielsweise, wie das erste Messverfahren, ein Streifenprojektionsverfahren jedoch unter Verwendung eines Projektionsgitters mit einer größeren Gitterperiode und ohne einer vorangehenden Puderung sein. Als das zweite Messverfahren können auch andere Verfahren, wie das konfokale Mikroskopieverfahren oder das Weißlichtinterferometrieverfahren verwendet werden. Die untere Zahnreihe 8 umfasst auch Nachbarzähne 12, 13. Bei der Planung der Restauration 11 werden die Form und die Ausrichtung der mittels des ersten Messverfahrens aufgenommenen Präparation 10 und der mittels des zweiten Messverfahrens aufgenommenen Nachbarzähne 12, 13 und der Gegenzähne 9 verwendet. Während der Vermessung des ersten Bereichs 4 mittels der ersten Vermessungsvorrichtung 5 werden erste Bilddaten 14 des ersten Bereichs erzeugt, die mittels der Anzeigevorrichtung 15 dargestellt sind. Bei der Vermessung des zweiten Bereichs 7 mittels der zweiten Vermessungsvorrichtung 6 werden zweite Bilddaten 16 erzeugt, die in Überlagerungen mit den ersten Bilddaten mittels der Anzeigevorrichtung 15 angezeigt werden. Die ersten Bilddaten 14 und die zweiten Bilddaten 16 werden an den Computer 17 übermittelt und zu einem dreidimensionalen Modell verrechnet, das mittels der Anzeigevorrichtung 15 aus unterschiedlichen Blickrichtungen betrachtet werden kann. Der erste Bereich 4 ist vom zweiten Bereich 8 des dentalen Objekts 1 umfasst, so dass die Präparationssehnen sowohl mittels der ersten Messvorrichtung 5 als auch mittels der zweiten Messvorrichtung 6 vermessen werden. Durch Vergleich der ersten Bilddaten 14 und der zweiten Bilddaten 16 können Aufnahmefehler im Bereich der Präparation festgestellt und korrigiert werden. Die Korrektur kann automatisch unter Verwendung bekannter Mustererkennungsverfahren oder manuell durch den Benutzer mittels der Eingabemittel 18 und 19 korrigiert werden. Der Aufnahmefehler 20 kann beispielsweise eine Erhöhung sein, die durch mangelnde Puderung und damit durch überhöhte Reflexionen hervorgerufen wird. Dieser Aufnahmefehler 20 kann mittels der Mustererkennungsverfahren erkannt werden oder manuell mittels der Eingabemittel 18, 19 markiert werden. Die ersten Bilddaten 14 des ersten Bereichs und die zweiten Bilddaten 16 des zweiten Bereichs 7 werden zu einer überlagerten dreidimensionalen Aufnahme 21 zusammengeführt, die mittels der Anzeigevorrichtung 15 angezeigt wird.

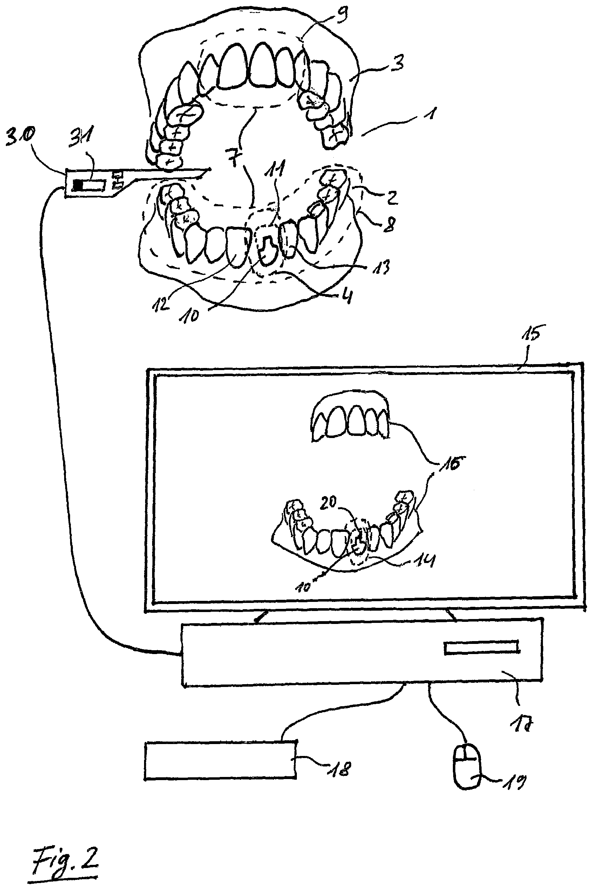

Die Fig. 2 zeigt eine Vorrichtung zur Durchführung eines alternativen Verfahrens, wobei im Vergleich zur Vorrichtung aus Fig.1 eine einzelne Messvorrichtung 30 verwendet wird, die einen Schalter 31 aufweist. In einem ersten Modus der Messvorrichtung 30 wird das erste präzise Messverfahren verwendet und in einem zweiten Modus wird das zweite weniger präzise Messverfahren verwendet. Im ersten Modus kann das Projektionsgitter mit einer kleineren Gitterperiode in den Strahlengang der Messvorrichtung 30 eingebracht werden, wobei eine vorangehende Puderung des ersten Bereichs 4 erforderlich ist. Im zweiten Modus wird ein Projektionsgitter mit einer größeren Gitterperiode im Strahlengang der Messvorrichtung 30 angebracht, so dass die Vermessung im zweiten Modus auch ohne vorangehende Puderung erfolgen kann. Im ersten Schritt wird die Messvorrichtung im ersten Modus zur Vermessung des ersten Bereichs 4 verwendet. Im zweiten Schritt wird die Messvorrichtung in den zweiten Modus umgeschaltet und zur Vermessung des zweiten Bereichs 7 des dentalen Objekts 1 verwendet.

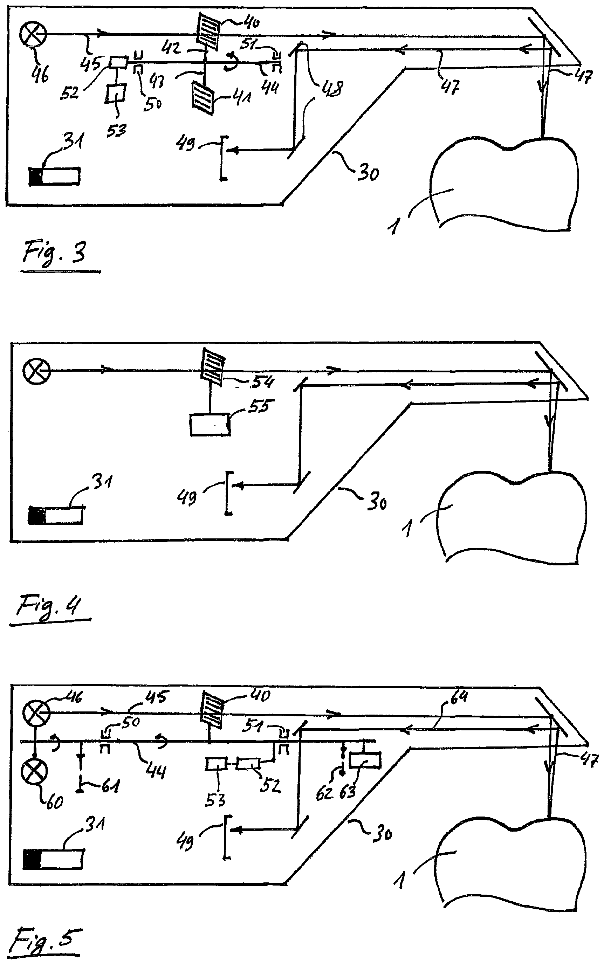

Die Fig. 3 zeigt eine Form der Vermessungsvorrichtung 30 aus Fig. 2, die ein erstes Projektionsgitter 40 mit einer ersten Gitterkonstante von 350 mm und ein zweites Projektionsgitter 41 mit einer größeren Gitterkonstanten von 700 mm aufweist. Das erste Projektionsgitter 40 und das zweite Projektionsgitter 41 sind schwenkbar über die Stege 42 und 43 mit einer Schwenkachse 44 verbunden. Die Schwenkachse 44 ist parallel zu einem Strahlengang 45 eines von einer ersten Lichtquelle 46 ausgehenden Beleuchtungsstrahls angeordnet. Bei betätigen des Schalters 31 wird die Vermessungsvorrichtung 30 auf den ersten Modus in den zweiten Modus umgeschaltet. Dabei wird die Schwenkachse 44 um 180° gedreht, so dass das zweite Gitter 41 im Strahlengang 45 des Beleuchtungsstrahls positioniert wird. Dadurch kann nun im zweiten Modus unter Verwendung des zweiten Verfahrens mit der größeren Gitterkonstante das dentale Objekt 1, wie ein einzelner Zahn oder eine Gruppe von Zähnen, vermessen werden. Beim Umschalten aus dem zweiten Modus in den ersten Modus wird die Schwenkachse 44 nochmals um 180° gedreht, so dass das erste Projektionsgitter 40 wieder im Strahlengang 45 positioniert wird. Der vom dentalen Objekt 1 zurückgestreute Beobachtungsstrahl 47 wird über zwei Strahlumlenker 48 zu einem Detektor 49 umgelenkt. Aus den erzeugten Bilddaten wird dann anschließend eine dreidimensionale Aufnahme des dentalen Objekts 1 erzeugt. Die Schwenkachse 44 ist in einem ersten Lager 50 und einem zweiten Lager 51 drehbar gelagert, wobei die Lager 50 und 51 Kugellager sein können. Die Schwenkachse 44 ist mittels eines Elektromotors 52, wie eines Schrittmotors, angetrieben, der mittels einer Motorsteuerung 53 entsprechend beim Betätigen des Schalters 31 angesteuert wird.

Die Fig. 4 zeigt eine weitere Form der Vermessungsvorrichtung 30 aus Fig. 2, wobei diese Form sich von der in Fig. 3 dargestellten Form dadurch unterscheidet, dass das erste und das zweite Projektionsgitter durch einen digitalen Lichtprojektor 54 erzeugt werden, der durch eine Steuerungsvorrichtung 55 entsprechend angesteuert wird, wenn der Schalter 31 in den ersten Modus oder in den zweiten Modus geschalten wird. Der digitale Lichtprojektor 54 kann aus Flüssigkristallelementen (LCD) aufgebaut sein.

Die Fig. 5 zeigt eine weitere Form der Vermessungsvorrichtung 30 aus Fig. 2, wobei das zweite Verfahren ein konfokales Mikroskopieverfahren ist. Auf der ersten oberen Seite der Schwenkachse 44 sind die ersten Mittel für den ersten Modus und auf der zweiten unteren Seite der Schwenkachse 44 sind die zweiten Mittel für den zweiten Modus angeordnet. Beim Umschalten der Vermessungsvorrichtung 30 vom ersten Modus in den zweiten Modus wird die Schwenkachse 44 um 180° gedreht, so dass die zweiten Mittel in den Strahlengang 45 der Vermessungsvorrichtung 30 gelangen. Die ersten Mittel für den ersten Modus umfassen eine erste Lichtquelle 46 und das erste Projektionsgitter 40. Die zweiten Mittel umfassen eine zweite Lichtquelle 60 eine erste Lochblende 61, eine zweite Lochblende 62 und ein Fokussiermittel 63. Die zweite Lichtquelle 60 kann eine monochromatische Lichtquelle wie ein Laser sein. Beim Umschalten in den zweiten Modus durch die Drehung der Schwenkachse 44 um 180° gelangt die zweite Lichtquelle 60 an die Stelle der ersten Lichtquelle 46, die erste Lochblende 61 gelangt in den Strahlengang 41, die zweite Lochblende und das Fokussiermittel 63 gelangen in einen Strahlengang 64 des Beobachtungsstrahls 47. Dadurch wird im zweiten Modus die Vermessung des dentalen Objekts 1 unter Verwendung des konfokalen Mikroskopieverfahrens ermöglicht. Die Schwenkachse 44 ist, wie die erste Ausführungsform aus Fig. 3, in einem ersten Lager 50 und einem zweiten Lager 51 drehbar gelagert, wobei die Lager 50 und 51 Kugellager sein können. Die Schwenkachse 44 ist mittels eines Elektromotors 52, wie eines Schrittmotors, angetrieben, der mittels einer Motorsteuerung 53 entsprechend angesteuert wird.

## Patentansprüche

1. Verfahren zur optischen dreidimensionalen Vermessung eines dentalen Objekts (1), wobei unter Verwendung eines ersten optischen dreidimensionalen Messverfahrens ein erster Bereich (4) des dentalen Objekts (1) vermessen wird, wobei das erste optische dreidimensionale Messverfahrens auf einem Triangulationsverfahren und auf einem Streifenprojektionsverfahren beruht, wobei an dem ersten Bereich (4) eine Puderung erfolgt, wobei erste Bilddaten (14) erzeugt werden, **dadurch gekennzeichnet, dass** unter Verwendung eines zweiten optischen dreidimensionalen Messverfahrens, das weniger präzise als das erste optische dreidimensionale Messverfahren ist, ein zweiter Bereich (7) des dentalen Objekts (1) ohne vorangegangene Puderung vermessen wird, wobei zweite Bilddaten (16) erzeugt werden, wobei die ersten Bilddaten (14) mit den zweiten Bilddaten (16) zu einer überlagerten dreidimensionalen Aufnahme (21) zusammengeführt werden.

2. Verfahren nach Anspruch 1, **dadurch gekennzeichnet, dass** das zweite optische dreidimensionale Messverfahren dem ersten optischen dreidimensionalen Messverfahren entspricht mit dem Unterschied, dass eine Gitterperiode eines Projektionsgitters größer ist und keine Puderung des zweiten Bereichs (7) erfolgt.

3. Verfahren nach einem der Ansprüche 1 oder 2, **dadurch gekennzeichnet, dass** das zweite optische dreidimensionale Messverfahren auf einem konfokalen Mikroskopieverfahren beruht.

4. Verfahren nach einem der Ansprüche 1 bis 3, **dadurch gekennzeichnet, dass** das zweite optische dreidimensionale Messverfahren auf einem Weisslichtinterferometrieverfahren beruht.

5. Verfahren nach einem der Ansprüche 1 bis 4, **dadurch gekennzeichnet, dass** das zweite optische dreidimensionale Messverfahren auf einem Triangulationsverfahren mit farblichen Mustern beruht.

6. Verfahren nach einem der Ansprüche 1 bis 5, **dadurch gekennzeichnet, dass** bei Durchführung des zweiten optischen dreidimensionalen Messverfahrens Farbinformationen des dentalen Objekts (1) erzeugt werden.

7. Verfahren nach einem der Ansprüche 1 bis 6, **dadurch gekennzeichnet, dass** der erste Bereich des dentalen Objekts eine Präparationsstelle (10) in der Mundhöhle eines Patienten ist.

8. Verfahren nach einem der Ansprüche 1 bis 7, **dadurch gekennzeichnet, dass** der zweite Bereich des dentalen Objekts einen Gegenzahn (9), einen zum ersten Bereich (4) benachbarten Nachbarzahn (12, 13) oder einen gesamten Kiefer (2) umfasst.

9. Verfahren nach einem der Ansprüche 1 bis 8, **dadurch gekennzeichnet, dass** der zweite Bereich (7) den ersten Bereich (4) des dentalen Objekts (1) umfasst.

10. Verfahren nach einem der Ansprüche 1 bis 9, **dadurch gekennzeichnet, dass** die ersten Bilddaten (14) mit den zweiten Bilddaten (16) verglichen werden und dadurch fehlerhafte Stellen in den ersten Bilddaten (14) erkannt werden.

11. Verfahren nach einem der Ansprüche 1 bis 10, **dadurch gekennzeichnet, dass** die fehlerhaften Stellen in den ersten Bilddaten (14) korrigiert werden.

12. Verfahren nach einem der Ansprüche 1 bis 11, **dadurch gekennzeichnet, dass** der zweite Bereich (7) und der erste Bereich (4) sich nicht überschneiden.

## Claims

1. A method for the optical three-dimensional measurement of a dental object (1), a first region (4) of the dental object (1) being measured using a first optical three-dimensional measurement method, the first optical three-dimensional measurement method being based on a triangulation method and on a strip projection method, the first region (4) being powdered, first image data (14) being generated, **characterised in that** a second region (7) of the dental object (1) is measured using a second optical three-dimensional measurement method, which is less precise than the first optical three-dimensional measurement method, without prior powdering, second image data (16) being generated, the first image data (14) being combined with the second image data (16) to form a superimposed three-dimensional image (21).

2. The method according to claim 1, **characterised in that** the second optical three-dimensional measurement method corresponds to the first optical three-dimensional measurement method, with the difference that a grating period of a projection grating is larger and the second region (7) is not powdered.

3. The method according to either claim 1 or claim 2, **characterised in that** the second optical three-dimensional measurement method is based on a confocal microscopy method.

4. The method according to any one of claims 1 to 3, **characterised in that** the second optical three-dimensional measurement method is based on a white light interferometry method.

5. The method according to any one of claims 1 to 4, **characterised in that** the second optical three-dimensional measurement method is based on a triangulation method with colour patterns.

6. The method according to any one of claims 1 to 5, **characterised in that** colour information about the dental object (1) is generated when the second optical three-dimensional measurement method is carried out.

7. The method according to any one of claims 1 to 6, **characterised in that** the first region of the dental object is a preparation site (10) in the oral cavity of a patient.

8. The method according to any one of claims 1 to 7, **characterised in that** the second region of the dental object comprises an opposing tooth (9), a neighbouring tooth (12, 13) adjacent to the first region (4), or an entire jaw (2).

9. The method according to any one of claims 1 to 8, **characterised in that** the second region (7) comprises the first region (4) of the dental object (1).

10. The method according to any one of claims 1 to 9, **characterised in that** the first image data (14) are compared with the second image data (16) and as a result faulty locations in the first image data (14) are detected.

11. The method according to any one of claims 1 to 10, **characterised in that** the faulty locations in the first image data (14) are corrected.

12. The method according to any one of claims 1 to 11, **characterised in that** the second region (7) and the first region (4) do not overlap.

## Revendications

1. Procédé de mesure optique tridimensionnelle d'un objet (1) dentaire, dans lequel, à l'aide d'un premier procédé de mesure optique tridimensionnelle, une première zone (4) de l'objet (1) dentaire est mesurée, dans lequel le premier procédé de mesure optique tridimensionnelle repose sur un procédé de triangulation et sur un procédé de projection de lumière structurée, dans lequel dans la première zone (4) un poudrage est effectué, dans lequel des premières données d'image (14) sont générées, **caractérisé en ce que,** à l'aide d'un second procédé de mesure optique tridimensionnelle, lequel est moins précis que le premier procédé de mesure optique tridimensionnelle de l'objet (1) dentaire, une seconde zone (7) est mesurée sans poudrage préalable, dans lequel des secondes données d'image (16) sont générées, dans lequel les premières données d'image (14) sont combinées avec les secondes données d'image (16) pour former une image (21) tridimensionnelle superposée.

2. Procédé selon la revendication 1, **caractérisé en ce que** le second procédé de mesure optique tridimensionnelle correspond au premier procédé de mesure optique tridimensionnelle, à la différence qu'une période de grille d'une grille de projection est supérieure et que dans la seconde zone (7) aucun poudrage n'est effectué.

3. Procédé selon l'une quelconque des revendications 1 ou 2, **caractérisé en ce que** le second procédé de mesure optique tridimensionnelle repose sur un procédé de microscopie confocale.

4. Procédé selon l'une quelconque des revendications 1 à 3, **caractérisé en ce que** le second procédé de mesure optique tridimensionnelle repose sur un procédé d'interférométrie en lumière blanche.

5. Procédé selon l'une quelconque des revendications 1 à 4, **caractérisé en ce que** le second procédé de mesure optique tridimensionnelle repose sur un procédé de triangulation avec des motifs colorés.

6. Procédé selon l'une quelconque des revendications 1 à 5, **caractérisé en ce que**, lors de la mise en œuvre du second procédé de mesure optique tridimensionnelle, des informations de couleur de l'objet (1) dentaire sont générées.

7. Procédé selon l'une quelconque des revendications 1 à 6, **caractérisé en ce que** la première zone de l'objet dentaire est un site de préparation (10) dans la cavité buccale d'un patient.

8. Procédé selon l'une quelconque des revendications 1 à 7, **caractérisé en ce que** la seconde zone de l'objet dentaire comprend une dent antagoniste (9), une dent voisine (12, 13) adjacente à la première zone (4) ou une mâchoire (2) entière.

9. Procédé selon l'une quelconque des revendications 1 à 8, **caractérisé en ce que** la seconde zone (7) comprend la première zone (4) de l'objet (1) dentaire.

10. Procédé selon l'une quelconque des revendications 1 à 9, **caractérisé en ce que** les premières données d'image (14) sont comparées aux secondes données d'image (16) et en conséquence des emplacements défectueux dans les premières données d'image (14) sont détectés.

11. Procédé selon l'une quelconque des revendications 1 à 10, **caractérisé en ce que** les emplacements défectueux dans les premières données d'image (14) sont corrigés.

12. Procédé selon l'une quelconque des revendications 1 à 11, **caractérisé en ce que** la seconde zone (7) et la première zone (4) ne se chevauchent pas.
